# EUROPEAN PATENT APPLICATION

(11) **EP 4 364 637 A1**
(43) Date of publication of application: **08.05.2024**
(21) Application number: 22832655.9
(22) Date of filing: 25.05.2022
(51) Int. Cl.: A61B 1/06

(54) **PROCESSOR FOR ENDOSCOPE AND ENDOSCOPE SYSTEM**

(30) Priority: 30.06.2021 JP 2021108566
(71) Applicant: HOYA CORPORATION, Shinjuku-ku Tokyo 160-8347 (JP)
(72) Inventor: YAMABE Toshiaki, Tokyo 160-8347 (JP)
(74) Representative: Schaumburg und Partner Patentanwälte mbB
(86) International application number: PCT/JP2022/021339
(87) International publication number: WO 2023/276497

(57) **Abstract**

One embodiment of the present invention is a processor for an endoscope configured to process a captured image of a biological tissue. This processor for an endoscope includes: a light source device that emits a light for illuminating a biological tissue; a spectrum sensor that acquires an optical spectrum of an emitted light from the light source device; and an adjustment unit that adjusts the optical spectrum of the emitted light from the light source device so that a difference between the optical spectrum acquired by the spectrum sensor and a predetermined target spectrum is reduced.

## Description

### Technical Field

The present invention relates to a processor for an endoscope and an endoscope system provided with a light source device.

### Background Art

In a medical equipment field, there is a known endoscope system capable of generating an image suitable for diagnosing a lesioned part hidden in a body cavity, by illuminating a biological tissue in the body cavity and imaging the illuminated biological tissue in the body cavity as an object. Conventionally, a lamp light source such as a Xenon lamp or a halogen lamp that emits a white-color light has been used as an illumination light. However, recently, a semiconductor light source having a light emitting element such as a light emitting diode (LED) or a laser diode (LD) that emits a light having a specific wavelength bandwidth has been used instead of the lamp light source.

For example, Japanese Patent No. 6138203 discloses an endoscope device configured to obtain an illumination light by a plurality of LEDs. According to Japanese Patent No. 6138203, this endoscope device is provided with a plurality of optical sensors respectively corresponding to the plurality of LEDs, and corrects a light quantity ratio among the LEDs on the basis of a detection result of each optical sensor, thereby obtaining an optimum light quantity ratio regardless of temperature characteristics of the LEDs.

### Summary of Invention

### Technical Problem

However, since the output characteristics of the LED change depending on not only the change in the light quantity due to the temperature characteristics, but also various variables such as light quantity attenuation (decrease) due to a deterioration with age, wavelength shift due to an increase in drive current, and a color balance in wavelength of each LED, it is not easy to adjust (correct) the illumination light. It is difficult to prepare the output characteristics of the LEDs corresponding to all of these variables in advance as a data table, and the flexibility is poor.

In another point of view, in a case of using a wide field of view (WFOV) sensor scope having an image sensor in which high-definition pixels are arranged, it is assumed that digital zoom is often used. In that case, the distance to an observation site tends to be relatively long. In this case, it is assumed that the light source output is increased in order to prevent the darkening of the captured image. However, the optical spectrum of the irradiation light changes in accordance with the increase in the light source output, and the optical balance may be affected.

Thus, the present invention aims to provide a processor for an endoscope and an endoscope system capable of easily suppressing the fluctuation of optical balance in an illumination light when a biological tissue in a body cavity is illuminated.

### Solution to Problem

One aspect of the present invention is a processor for an endoscope configured to process a captured image of a biological tissue. This processor for an endoscope includes:
a light source device that emits a light for illuminating a biological tissue;
a spectrum sensor that acquires an optical spectrum of an emitted light from the light source device; and
an adjustment unit that adjusts the optical spectrum of the emitted light from the light source device so that a difference between the optical spectrum acquired by the spectrum sensor and a predetermined target spectrum is reduced.

The light source device may include a UV light emitting element that emits a light having a wavelength of an ultraviolet ray.

In that case, the processor for an endoscope may include a temperature control unit that controls a temperature of the UV light emitting element so that a light intensity of an emitted light from the UV light emitting element does not exceed a predetermined peak value.

The light source device may include a plurality of light emitting elements that emit lights having different wavebands.

In that case, the adjustment unit may acquire wavelength shift quantities of the plurality of light emitting elements based on a comparison result between the optical spectrum acquired by the spectrum sensor and the predetermined target spectrum, and may adjust the optical spectrum of the emitted light from the light source device in a case where the wavelength shift quantity of at least one of the plurality of light emitting elements exceeds a predetermined value.

An optical fiber that is disposed between a light emitting end of the light source device and the spectrum sensor and that guides a part of the emitted light from the light source device through the light emitting end to the spectrum sensor, may be provided.

In that case, a fiber Bragg grating (FBG) may be disposed in the optical fiber,
the spectrum sensor may detect a wavelength of a reflected light reflected by the FBG out of the emitted light from the light source device, and
the control unit may correct the optical spectrum acquired by the spectrum sensor based on the wavelength of the reflected light detected by the spectrum sensor.

Another aspect of the present invention is an endoscope system including the processor for an endoscope, and an endoscope scope connected to the processor for an endoscope. The endoscope scope includes:
a second light source device that emits a light for illuminating the biological tissue; and
an optical coupling unit that couples an emitted light from a first light source device that is a light source device provided in the processor for an endoscope and an emitted light from the second light source device.

The endoscope scope may include a second spectrum sensor that acquires an optical spectrum of a coupled light coupled by the optical coupling unit.

In that case, the adjustment unit may adjust an optical spectrum of the emitted light from the first light source device and/or the second light source device based on a first optical spectrum acquired by the spectrum sensor that is provided in the processor for an endoscope and a second optical spectrum acquired by the second spectrum sensor so that a difference between the second optical spectrum and the target spectrum becomes small.

Still another aspect of the present invention is an endoscope system including a processor for an endoscope, and an endoscope scope connected to the processor for an endoscope.

The processor for an endoscope includes a light source device that emits a light for illuminating a biological tissue, and
a spectrum sensor that acquires an optical spectrum of an emitted light from the light source device.

The processor for an endoscope further includes an adjustment unit that adjusts the optical spectrum of the emitted light from the light source device so that a difference between the optical spectrum acquired by the spectrum sensor and a predetermined target spectrum is reduced.

### Advantageous Effects of Invention

According to the processor for an endoscope and the endoscope system described above, it is possible to easily suppress the fluctuation of optical balance in an illumination light when the biological tissue in a body cavity is illuminated.

### Brief Description of Drawings

Fig. 1 is a block diagram illustrating an example of a configuration of an endoscope system according to one embodiment.
Fig. 2 is a block diagram illustrating an example of a configuration of a light source device according to the embodiment.
Fig. 3 is a block diagram illustrating an example of a configuration of a light source device according to an embodiment different from Fig. 2.
Fig. 4 is an example of a flowchart illustrating adjustment processing of an LED light source in an electronic endoscope system of the embodiment illustrated in Fig. 2 or 3.
Fig. 5 is a block diagram illustrating an example of a configuration of a light source device according to an embodiment different from Fig. 3.
Fig. 6 is a block diagram illustrating an example of a configuration of a light source device according to an embodiment different from Fig. 5.
Fig. 7 is an example of a flowchart illustrating adjustment processing of an LED light source in an electronic endoscope system of the embodiment illustrated in Fig. 6.

### Description of Embodiments

Hereinafter, an electronic endoscope system will be described in detail with reference to the accompanying drawings.

A processor for an electronic endoscope according to one embodiment includes a light source device that emits a light for illuminating a biological tissue, a spectrum sensor, and an adjustment unit that adjusts an optical spectrum of an emitted light from the light source device. A light source in the light source device is not limited, but is a light emitting element such as a light emitting diode and a laser diode.

In an electronic endoscope system according to the embodiment, the spectrum sensor acquires the optical spectrum of the emitted light from the light source device. The adjustment unit is configured to adjust the optical spectrum of the emitted light from the light source device so that a difference between the optical spectrum acquired by the spectrum sensor and a predetermined target spectrum is reduced. Therefore, it is possible to easily suppress the fluctuation of optical balance in an illumination light when the biological tissue in a body cavity is illuminated, and thus, it is advantageous particularly in a case of using a wide field of view (WFOV) sensor scope having an image sensor in which high-definition pixels are arranged. That is, even in a situation where the light source output increases due to a distance from the sensor scope to an observation site becoming relatively large, by using the digital zoom, the change in the optical spectrum of an irradiation light in accordance with the increase in the light source output is suppressed.

In one embodiment, the spectrum sensor is not limited to a case of being included in a processor for an electronic endoscope. Since it is sufficient that the optical spectrum of the illumination light can be monitored before the illumination light emitted from the light source device reaches the biological tissue, the spectrum sensor may be provided in an electronic scope. Even in this case, the optical spectrum of the emitted light from the light source device can be adjusted by performing data processing on the optical spectrum acquired by the spectrum sensor by the adjustment unit in the processor for an electronic endoscope.

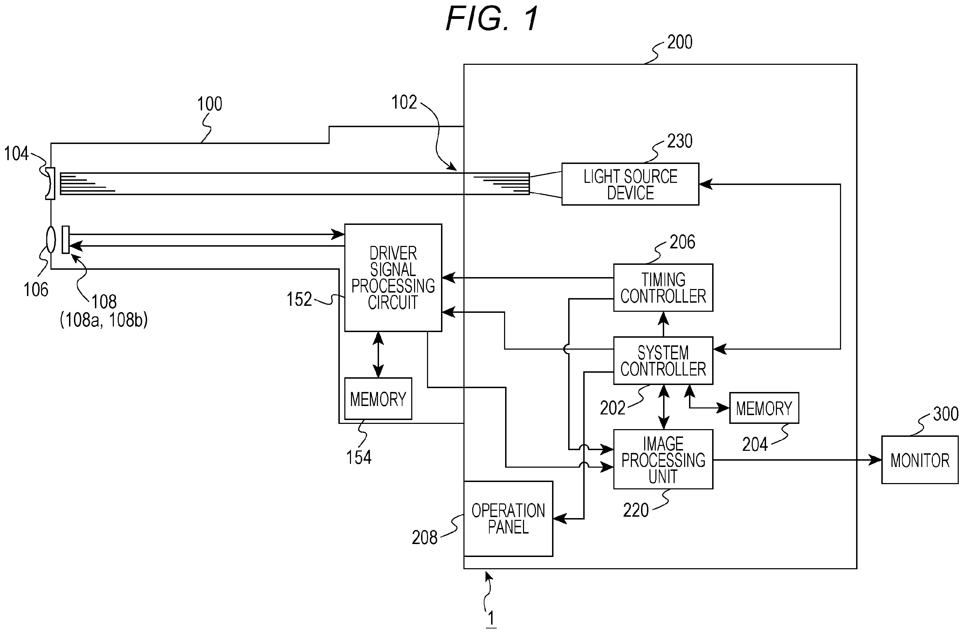

Fig. 1 is a block diagram illustrating an example of a configuration of an electronic endoscope system 1 according to the embodiment. As illustrated in Fig. 1, the electronic endoscope system 1 includes an electronic scope 100, a processor 200 for an electronic endoscope, and a monitor 300.

The processor 200 for an electronic endoscope includes a system controller 202 and a timing controller 206. The system controller 202 executes various programs stored in a memory 204 and integrally controls the entire electronic endoscope system 1. In addition, the system controller 202 changes various settings of the electronic endoscope system 1 according to an instruction from a user (operator or assistant) input to an operation panel 208. The timing controller 206 outputs a clock pulse for adjusting operation timing of each unit to each circuit in the electronic endoscope system 1.

The processor 200 for an electronic endoscope includes a light source device 230 that supplies an illumination light to the electronic scope 100. The light source device 230 is configured so that the illumination light is collected by a condensing lens (not illustrated), and thereafter, is incident on an incident end of a light carrying bundle (LCB) 102 which is a bundle of optical fiber strands of the electronic scope 100 via a dimmer (not illustrated).

The light source device 230 includes a light source (hereinafter, referred to as an "LED light source" as appropriate) including a plurality of light emitting diodes each emitting a light having a wavelength bandwidth of a predetermined color. A laser diode can be used instead of the LED. The LED and the laser diode have features such as low power consumption, a low heat generation amount, and the like, as compared with other light sources, and thus, have an advantage that a bright image can be acquired while suppressing power consumption or a heat generation amount. Since a bright image can be acquired, it is possible to improve the accuracy in evaluation pertaining to the degree of lesion in a lesioned part.

Note that, in the example illustrated in Fig. 1, the light source device 230 is provided to be built in the processor 200 for an electronic endoscope, but may be provided in the electronic endoscope system 1 as a device separate from the processor 200 for an electronic endoscope. In addition, the light source device 230 may be provided in a distal tip of the electronic scope 100 to be described later. In this case, the LCB 102 that guides the illumination light is unnecessary.

The illumination light incident from the incident end into the LCB 102 transmits in the LCB 102 and is emitted from an emission end of the LCB 102 placed in the distal tip of the electronic scope 100, and is applied to an object via a light distribution lens 104. A reflected light from the object forms an optical image on a light receiving surface of an image sensor 108 via an objective lens 106.

The image sensor 108 is, for example, a single-chip color charge-coupled device (CCD) image sensor in which various filters of an infrared (IR) cut filter 108a and a Bayer array color filter 108b are arranged on a light receiving surface, and generates primary color signals of red (R), green (G), and blue (B) corresponding to an optical image formed on the light receiving surface. Instead of the single-plate color CCD image sensor, a single-plate color complementary metal oxide semiconductor (CMOS) image sensor can also be used.

A driver signal processing circuit 152 is provided in a connection portion of the electronic scope 100. The driver signal processing circuit 152 generates an image signal (brightness signal Y and color difference signal Cb, Cr) by performing predetermined signal processing such as color interpolation or a matrix calculation on the primary color signal input from the image sensor 108, and outputs the generated image signal to an image processing unit 220 of the processor 200 for an electronic endoscope. In addition, the driver signal processing circuit 152 accesses a memory 154, and reads specific information of the electronic scope 100. For example, the specific information of the electronic scope 100 recorded in the memory 154 includes the number of pixels or sensitivity of the image sensor 108, a frame rate with which the electronic scope 100 is operable, and a model number. The driver signal processing circuit 152 outputs the specific information read from the memory 154 to the system controller 202. In this way, the electronic scope 100 uses the image sensor 108 to image a biological tissue inside a body cavity.

The system controller 202 performs various calculations, based on the specific information of the electronic scope 100, and generates a control signal. The system controller 202 controls an operation and a timing of each circuit inside the processor 200 for an electronic endoscope by using the generated control signal so that processing suitable for the electronic scope 100 connected to the processor 200 for an electronic endoscope is performed.

The timing controller 206 generates a timing signal composed of a clock pulse in accordance with the timing control by the system controller 202, and provides the timing signal to the image sensor 108, the driver signal processing circuit 152, the image processing unit 220, and the light source device 230. The driver signal processing circuit 152 is driven in accordance with the clock pulse of the timing signal supplied from the timing controller 206.

Under the control of the system controller 202, the image processing unit 220 generates a video signal for monitor display of an endoscope image or the like based on the image signal input from the driver signal processing circuit 152, and outputs the video signal to the monitor 300 in accordance with the timing signal.

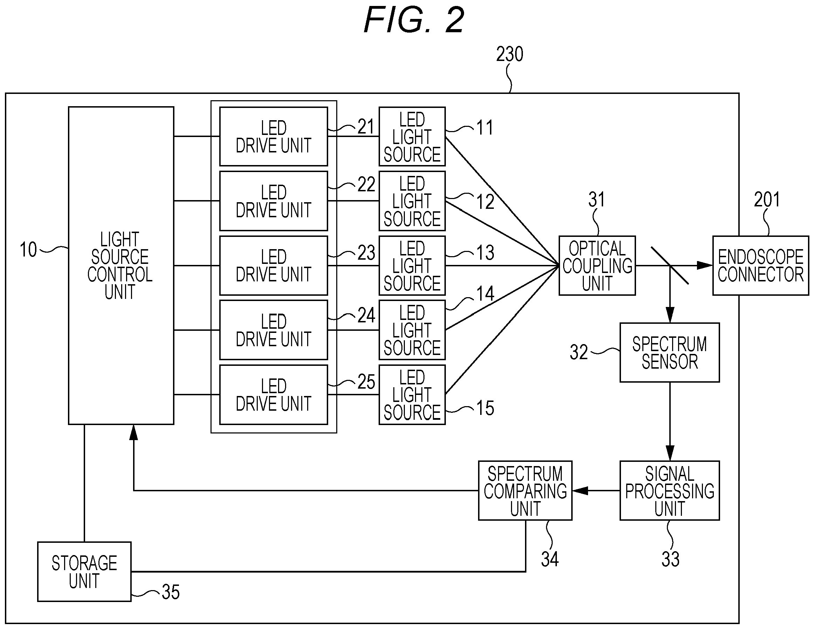

A block diagram of the light source device 230 is illustrated in Fig. 2. The light source device 230 includes a light source control unit 10, LED light sources 11-15, LED drive units 21-25, and an optical coupling unit 31.

The LED light sources 11-15 are individually controlled to emit lights by the LED drive units 21-25.

The LED light source 11 is an LED light source that emits a light having a wavelength bandwidth of an ultraviolet (UV) ray (for example, a wavelength of 375-435 nm; hereinafter, also referred to as "UV wavelength bandwidth"). The LED light source 12 is an LED light source that emits a light having a blue-color wavelength bandwidth (for example, a wavelength of 420-510 nm). The LED light source 13 is an LED light source that emits a light having a green-color wavelength bandwidth (for example, wavelength of 520-580 nm). The LED light source 14 is an LED light source that emits a light having an amber-color wavelength bandwidth (for example, a wavelength of 590-620 nm). The LED light source 15 is an LED light source that emits a light having a red-color wavelength bandwidth (for example, a wavelength of 630-680 nm).

Each emitted light from the LED light sources 11-15 is coupled by the optical coupling unit 31 and is incident on the LCB 102 via an endoscope connector 201.

The light source control unit 10 is configured by, for example, a central processing unit (CPU) or a field programmable gate array (FPGA), and is configured to substantially control the light intensity of the emitted light from each of the LED light sources 11-15 by transmitting a signal indicating the value of current caused to flow through the corresponding LED light source to the LED drive units 21-25.

As illustrated in Fig. 2, the light source device 230 further includes a spectrum sensor 32, a signal processing unit 33, a spectrum comparing unit 34, and a storage unit 35.

The spectrum sensor 32 acquires, from an emitted light from the optical coupling unit 31, an optical spectrum and a light quantity thereof. The spectrum sensor 32 performs a spectrum measurement to cover the wavelength bandwidth of each emitted light from the LED light sources 11-15. The spectrum sensor 32 includes, for example, a plurality of photodiodes corresponding to a plurality of channels (a plurality of divided wavelength bandwidths) that cover the entire wavelength bandwidth to be detected. Each of the plurality of photodiodes is provided with a filter that transmits therethrough a light having a wavelength bandwidth corresponding to each channel. The spectrum sensor 32 measures the optical spectrum of the emitted light from the optical coupling unit 31 on the basis of the detection value of each photodiode from the emitted light from the optical coupling unit 31.

In one embodiment, some of the plurality of channels of the spectrum sensor 32 correspond to the wavelength bandwidth of each emitted light from the LED light sources 11-15.

For example, the signal processing unit 33 performs predetermined signal processing on the optical spectrum (that is, a signal of the detection value of each channel) obtained by the spectrum sensor 32. For example, the signal processing unit 33 may perform signal processing (for example, normalization processing based on a signal of a specific channel, or the like) appropriate for performing comparison processing in the spectrum comparing unit 34.

The storage unit 35 is a nonvolatile memory, and stores data related to a target spectrum. The target spectrum may be an optical spectrum that serves as a predetermined target as the emitted light from the optical coupling unit 31, or may be, for example, an actual measurement value in an early stage of the optical spectrum of the emitted light from the optical coupling unit 31 measured at the time of manufacturing the processor 200 for an electronic endoscope.

In one embodiment, the target spectrum is data of a spectrum corresponding to each channel of the spectrum sensor 32.

The spectrum comparing unit 34 compares the optical spectrum of the emitted light from the optical coupling unit 31 obtained by the signal processing unit 33 with the target spectrum read from the storage unit 35, and sends data indicating the comparison result to the light source control unit 10.

Based on the comparison result in the spectrum comparing unit 34, the light source control unit 10 (one example of the adjustment unit) transmits a signal indicating the value of current caused to flow through the corresponding LED light source to the LED drive units 21-25 so that the difference between the optical spectrum of the emitted light from the optical coupling unit 31 and the target spectrum becomes small (for example, equal to or less than a predetermined threshold). Accordingly, the optical spectrum of the emitted light from the optical coupling unit 31 is adjusted to be close to the target spectrum.

The spectrum sensor 32 may not be provided in a processor for an electronic endoscope.

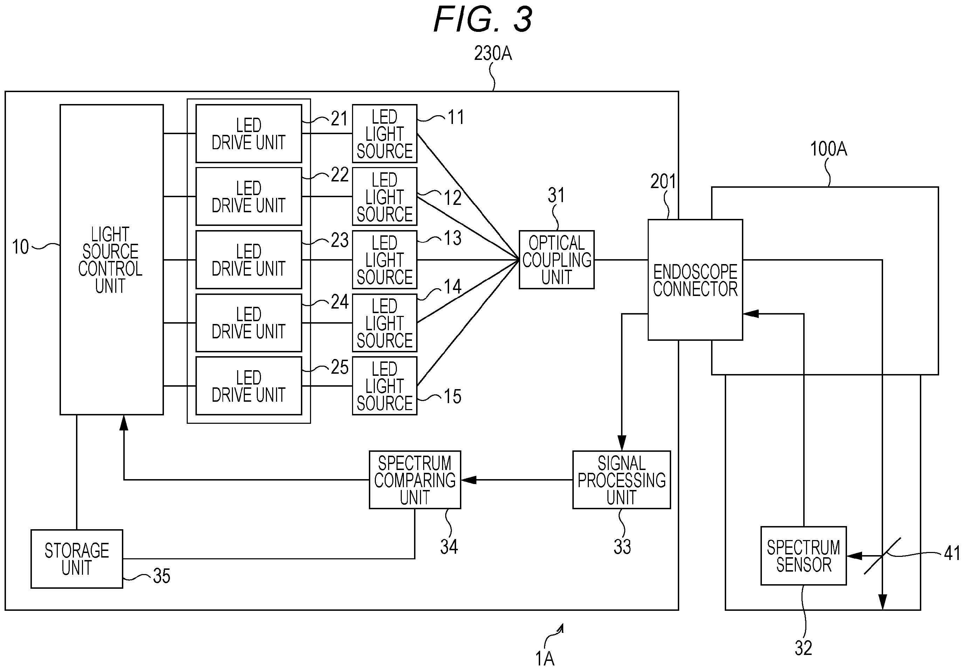

In an electronic endoscope system 1A according to one embodiment, as illustrated in Fig. 3, a light source device 230A of the processor 200 for an electronic endoscope includes no spectrum sensor 32, and a spectrum sensor 32 is provided in an electronic scope 100A. In this case, the emitted light emitted from the LCB 102 (not illustrated in Fig. 3) in the electronic scope 100A is incident on the spectrum sensor 32 through a reflecting surface 41, and the optical spectrum obtained by the spectrum sensor 32 is sent to the signal processing unit 33 of the light source device 230A via the endoscope connector 201.

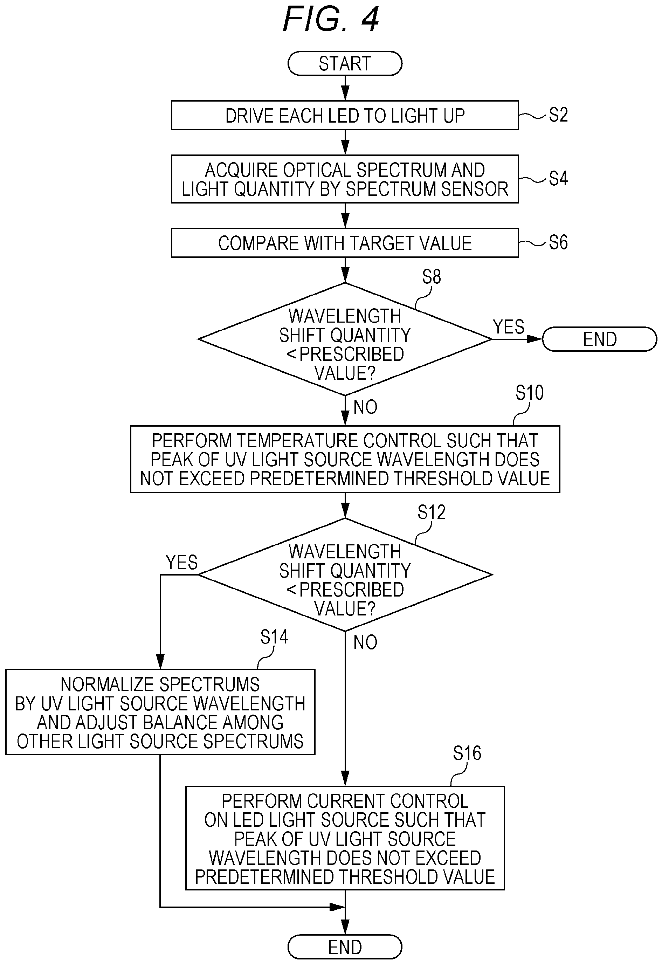

Next, with reference to a flowchart in Fig. 4, the adjustment processing of an LED light source executed in the electronic endoscope system will be described.

With reference to Fig. 4, the light source control unit 10 transmits a signal to the LED driving units 21-25, along with the activation of the processor 200 for an electronic endoscope, whereby each LED drive unit turns on the corresponding LED light source (step S2).

The optical coupling unit 31 performs coupling of emitted lights from the LED light sources 11-15, and the emitted light from the optical coupling unit 31 is incident on the LCB 102 to illuminate the biological tissue. The emitted light from the optical coupling unit 31 is also directed to the spectrum sensor 32. The spectrum sensor 32 acquires the optical spectrum and the light quantity of the emitted light from the optical coupling unit 31 (step S4).

The spectrum comparing unit 34 compares the optical spectrum obtained by the spectrum sensor 32 with the target spectrum read from the storage unit 35 (step S6), and sends data indicating the comparison result to the light source control unit 10.

In one embodiment, the data indicating the comparison result in the spectrum comparing unit 34 is data indicating a difference in peak wavelength per channel between the optical spectrum acquired by the spectrum sensor 32 and the target spectrum. For example, the peak wavelength of the optical spectrum of the channel corresponding to a light of the blue-color wavelength bandwidth (for example, a wavelength of 420-510 nm) among optical spectrums obtained from the spectrum sensor 32 is calculated, and a difference (wavelength shift quantity) between the peak wavelength and a peak wavelength (for example, 475 nm) of the blue-color wavelength bandwidth defined in the target spectrum is calculated.

Based on the data received from the spectrum comparing unit 34, the light source control unit 10 determines whether or not the wavelength shift quantity of a light having a wavelength bandwidth emitted from each of the LED light sources 11-15 is smaller than a prescribed value (step S8), and it is determined as normal in a case where the wavelength shift quantity is small and thus the process is ended with nothing to be performed.

On the other hand, in a case where the wavelength shift quantity is equal to or larger than the prescribed value (step S8: NO), the light source control unit 10 (one example of the temperature control unit) firstly performs temperature control so that the peak of the UV light source wavelength does not exceed a predetermined threshold (step S10). That is, since the peak of the UV light with high energy affects the heat of the distal end of the electronic scope 100, it is preferable to limit the peak of the UV light in consideration of blood coagulation and biological damage by the illumination light. Therefore, the light source control unit 10 controls the LED drive unit 21 corresponding to the LED light source 11 that emits the light having the UV wavelength so that the peak level of the UV wavelength bandwidth measured by the spectrum sensor 32 does not exceed the predetermined threshold. That is, the value of current flowing through the LED light source 11 is controlled.

Although not illustrated, in order to control the temperature of the LED light source, a temperature detector such as a thermistor and a fan motor for adjusting the temperature of the LED light source may be provided in the light source device 230.

Subsequently, based on the data received from the spectrum comparing unit 34, the light source control unit 10 determines again whether or not the wavelength shift quantity of a light having a wavelength bandwidth emitted from each of the LED light sources 11-15 is smaller than the prescribed value (step S12). In a case where the wavelength shift quantity is smaller than the prescribed value, the light source control unit 10 performs normalization (standardization) based on the peak level of the UV wavelength bandwidth measured by the spectrum sensor 32 and adjusts a balance among spectrums of the other wavelength bandwidths (step S14). For example, the light quantity of a light having a wavelength bandwidth other than the UV wavelength bandwidth is adjusted so that the emitted light from the optical coupling unit 31 becomes a white light which is a target.

On the other hand, in a case where the wavelength shift quantity is equal to or larger than the prescribed value (step S12: NO), the light source control unit 10 performs current control on the LED drive units 21-25 so that the peak of the UV light source wavelength does not exceed a predetermined threshold (step S16). Accordingly, it is possible to reduce the wavelength shift quantity of a light of each wavelength bandwidth, thereby bringing the optical spectrum of the emitted light from the optical coupling unit 31 close to the target spectrum. That is, the optical spectrum of a light emitted from the light source device 230 is adjusted.

Note that the storage unit 35 stores a lookup table (LUT) indicating the relation between the current and the wavelength of the emitted light for each LED light source, and the light source control unit 10 refers to the LUT so as to determine the value of current to be applied to each LED light source in order to reduce the wavelength shift quantity.

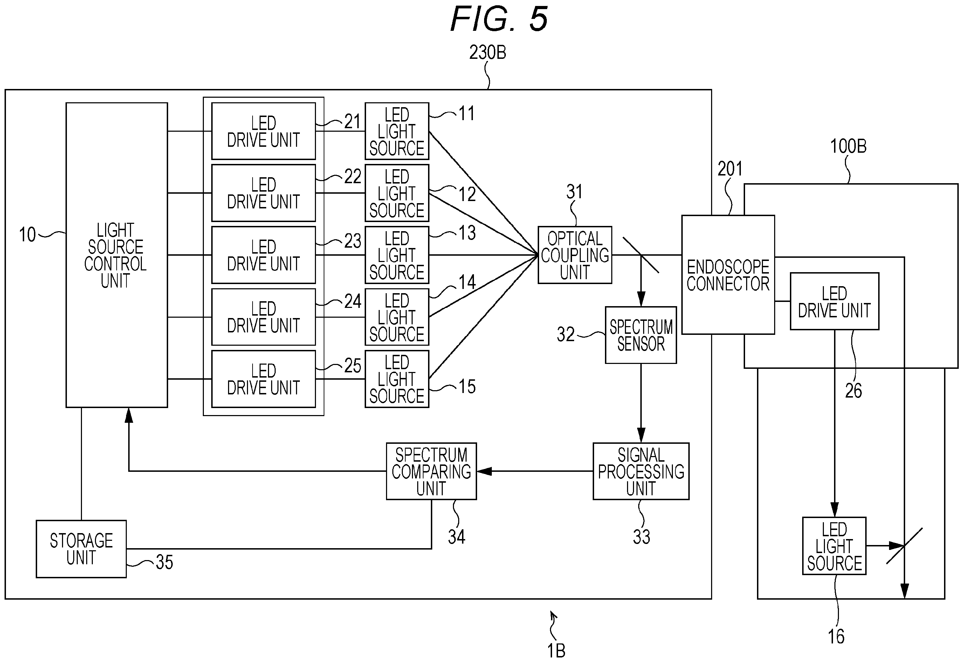

In one embodiment, as illustrated in Fig. 5, the electronic scope may be provided with an LED light source.

As illustrated in Fig. 5, an electronic endoscope system 1B according to one embodiment is provided with an electronic scope 100B including an LED drive unit 26 and an LED light source 16.

The light source control unit 10 of a light source device 230B is configured to substantially control the light intensity of an emitted light from the LED light source 16 by transmitting a signal indicating the value of current caused to flow through the corresponding LED light source to the LED drive unit 26. In an example illustrated in Fig. 5, the number of LED light sources provided in the electronic scope 100B is one, but the number is not limited and two or more LED light sources that emit lights having different wavelength bandwidths may be provided.

In the electronic endoscope system 1B, the emitted light from the optical coupling unit 31 of the light source device 230B (one example of the first light source device) and the emitted light from the LED light source 16 (one example of the second light source device) in the electronic scope 100B are coupled to each other to become an illumination light for the biological tissue in the body cavity.

By providing the LED light source 16 in the electronic scope 100B, there is an advantage that it is possible to complement a wavelength component of an emitted light (emitted light from the optical coupling unit 31) from the light source device 230B. For example, as illustrated in Fig. 4, in a case where it is difficult to reduce the wavelength shift quantity and adjust the balance among spectrums even after executing the adjustment processing on the LED light sources 11-15 in the light source device 230B, it is possible to perform complementation by using the emitted light from the LED light source 16 in the electronic scope 100B.

Although not illustrated, in one embodiment, an optical fiber including a fiber Bragg grating (FBG) may be provided between the light emitting end of the optical coupling unit 31 in the light source device 230 and the spectrum sensor 32. The optical fiber is configured to guide a part of the emitted light from the optical coupling unit 31 to the spectrum sensor 32.

The spectrum sensor 32 detects the wavelength of a reflected light reflected by the FBG by observing a spectrum of a transmitted light transmitting through the FBG out of the emitted light from the optical coupling unit 31. It is possible to arbitrarily set the Bragg wavelength in the FBG.

The light source control unit 10 corrects the optical spectrum acquired by the spectrum sensor 32 on the basis of the reflected light detected by the spectrum sensor 32, for example, on the basis of a wavelength shift quantity between the wavelength of the reflected light at reference temperature such as room temperature and the wavelength of the current reflected light during the operation. Therefore, the output fluctuation of the spectrum sensor 32 due to the temperature is corrected.

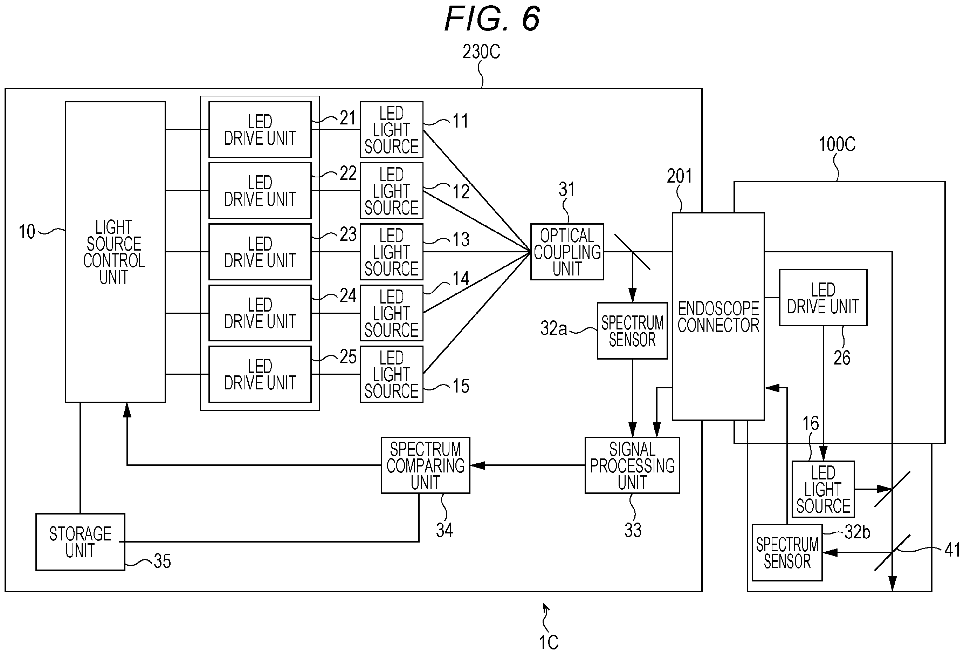

In one embodiment, a spectrum sensor may be provided not only on a side of the light source device in the processor for an electronic endoscope, but also on the electronic scope side. This embodiment is illustrated in Fig. 6.

In an electronic endoscope system 1C illustrated in Fig. 6, a spectrum sensor 32a is provided in a light source device 230C of the processor for an electronic endoscope, and a spectrum sensor 32b is provided in an electronic scope 100C.

The spectrum sensor 32a in the light source device 230C acquires the optical spectrum and the light quantity of the emitted light from the optical coupling unit 31 in the same manner as the embodiment illustrated in Fig. 2.

The electronic scope 100C is provided with an additional LED drive unit 26 and an additional LED light source 16 in the same manner as the embodiment illustrated in Fig. 5. The spectrum sensor 32b acquires the optical spectrum and the light quantity of the illumination light in which the emitted light from the optical coupling unit 31 and the emitted light from the LED light source 16 are combined.

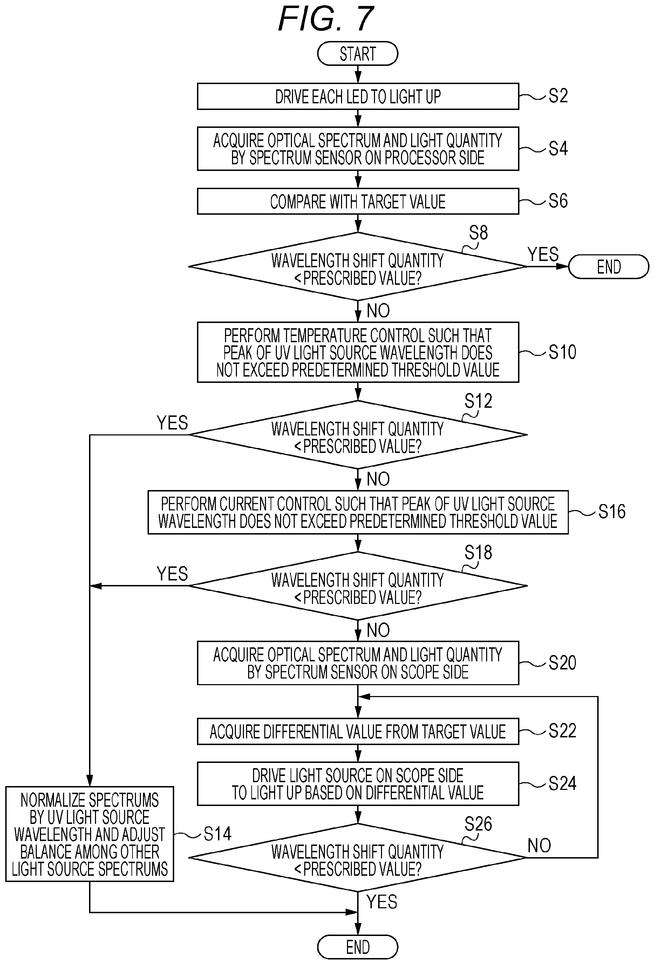

The adjustment processing of an LED light source executed in the electronic endoscope system 1C illustrated in Fig. 6 will be described with reference to a flowchart in Fig. 7.

Steps S2 to S16 in the flowchart in Fig. 7 indicate the adjustment processing of the LED light source in the light source device 230C, and are identical to steps S2 to S16 in Fig. 4, and thus redundant description will be omitted.

In the flowchart in Fig. 7, in a case where the wavelength shift quantity of the emitted light from the LED light sources 11-15 does not fall below the prescribed value even after the processing of steps S2 to S16 is executed (step S18: NO), the light source control unit 10 controls the LED light source 16 of the electronic scope 100C.

Specifically, the light source control unit 10 firstly acquires the optical spectrum and the light quantity of the illumination light by the spectrum sensor 32b on the electronic scope 100C side (step S20). Note that, at this point, the LED drive unit 26 does not drive the LED light source 16 to light up.

Next, the light source control unit 10 acquires a differential value between the target spectrum and the optical spectrum obtained by the spectrum sensor 32b on the scope side (step S22), and controls the LED drive unit 26 to drive the LED light source 16 that is the scope-side light source to light up on the basis of the differential value (step S34). That is, a portion that cannot be corrected by a light source device on the processor side is compensated by a light source device on the scope side (that is, the LED light source 16). Accordingly, the optical spectrum of the emitted light (that is, an illumination light to be applied on a biological tissue) from the electronic scope 100C is adjusted to be close to the target spectrum.

The configuration illustrated in Fig. 6 is also convenient when the calibration of an illumination light is performed during the manufacturing of the electronic endoscope system by connecting the processor for an electronic endoscope and the electronic scope. That is, in step S22, a specification value of the spectrum of the emitted light from the optical coupling unit 31 on the processor side and a differential value of the optical spectrum obtained by the spectrum sensor 32b on the scope side are acquired, and the LED light source 16 is driven to light up so that the difference value decreases. Accordingly, in a case where it is impossible to adapt to the specification value of the spectrum of the emitted light on the processor side alone, the complementation is performed on the scope side.

As described above, according to the electronic endoscope system of the embodiment, a spectrum sensor that acquires optical spectrum of an emitted light (illumination light) emitted from a light source device for illuminating a biological tissue is provided, and the optical spectrum of the emitted light from the light source device is adjusted so that the difference between the optical spectrum acquired by the spectrum sensor and the predetermined target spectrum is reduced, and thus, it is possible to suppress the fluctuation of optical balance in the illumination light as appropriate when the biological tissue in a body cavity are illuminated.

In particular, it is advantageous in a case of using a wide field of view (WFOV) sensor scope having an image sensor in which high-definition pixels are arranged. That is, even in a situation where the light source output increases due to a distance from the sensor scope to an observation site becoming relatively large, by using the digital zoom, the change in the optical spectrum of an irradiation light in accordance with the increase in the light source output is suppressed.

Hitherto, the electronic endoscope system of the present invention has been described in detail, but the present invention is not limited to the above-described embodiment. As a matter of course, various improvements or modifications may be made within the scope not departing from the concept of the present invention.

The present invention relates to a patent application of Japanese Patent Application No. 2021-108566 filed with the Japan Patent Office on June 30, 2021, the entire contents of which are incorporated herein by reference.

## Claims

1. A processor for an endoscope configured to process a captured image of a biological tissue, the processor for an endoscope comprising:
a light source device that emits a light for illuminating the biological tissue;
a spectrum sensor that acquires an optical spectrum of an emitted light from the light source device; and
an adjustment unit that adjusts the optical spectrum of the emitted light from the light source device so that a difference between the optical spectrum acquired by the spectrum sensor and a predetermined target spectrum is reduced.

2. The processor for an endoscope according to claim 1, wherein:
the light source device includes a UV light emitting element that emits a light having a wavelength of an ultraviolet ray; and
the processor for an endoscope includes a temperature control unit that controls a temperature of the UV light emitting element so that a light intensity of the emitted light from the UV light emitting element does not exceed a predetermined peak value.

3. The processor for an endoscope according to claim 1 or 2, wherein:
the light source device includes a plurality of light emitting elements that emit lights having different wavebands; and
the adjustment unit acquires wavelength shift quantities of the plurality of light emitting elements based on a comparison result between the optical spectrum acquired by the spectrum sensor and the predetermined target spectrum, and adjusts the optical spectrum of the emitted light from the light source device in a case where the wavelength shift quantity of at least one of the plurality of light emitting elements exceeds a predetermined value.

4. The processor for an endoscope according to any one of claims 1-3, wherein:
an optical fiber that is disposed between a light emitting end of the light source device and the spectrum sensor and that guides a part of the emitted light from the light source device through the light emitting end to the spectrum sensor, is provided;
a fiber Bragg grating (FBG) is disposed in the optical fiber;
the spectrum sensor detects a wavelength of a reflected light reflected by the FBG out of the emitted light from the light source device; and
the control unit corrects the optical spectrum acquired by the spectrum sensor based on the wavelength of the reflected light detected by the spectrum sensor.

5. An endoscope system comprising: the processor for an endoscope according to any one of claims 1-4; and an endoscope scope connected to the processor for an endoscope, wherein
the endoscope scope includes:
a second light source device that emits a light for illuminating the biological tissue; and
an optical coupling unit that couples an emitted light from a first light source device that is a light source device provided in the processor for an endoscope and an emitted light from the second light source device.

6. An endoscope system according to claim 5, wherein:
the endoscope scope includes a second spectrum sensor that acquires an optical spectrum of a coupled light coupled by the optical coupling unit, and
the adjustment unit adjusts an optical spectrum of the emitted light from the first light source device and/or the second light source device based on a first optical spectrum acquired by the spectrum sensor and a second optical spectrum acquired by the second spectrum sensor that are provided in the processor for an endoscope so that a difference between the second optical spectrum and the target spectrum becomes small.

7. An endoscope system comprising a processor for an endoscope, and an endoscope scope connected to the processor for an endoscope, wherein:
the processor for an endoscope includes a light source device that emits a light for illuminating the biological tissue;
the endoscope scope includes a spectrum sensor that acquires an optical spectrum of an emitted light from the light source device; and
the processor for an endoscope further includes
an adjustment unit that adjusts the optical spectrum of the emitted light from the light source device so that a difference between the optical spectrum acquired by the spectrum sensor and a predetermined target spectrum is reduced.
